(19) Europäisches Patentamt
European Patent Office
Office européen des brevets

(11) Publication number : 0 158 843 B1

# (12) EUROPEAN PATENT SPECIFICATION

(45) Date of publication of patent specification : 11.11.87

(51) Int. Cl.⁴ : C 07 D401/12, A 61 K 31/44

(21) Application number : 85103105.4

(22) Date of filing : 18.03.85

## (54) A N-(6-substituted pyridine-2-carbonyl)-3-substituted histidine derivative, a method of preparing the same and its use as an anti-tumor agent.

(30) Priority : 26.03.84 JP 56138/84

(43) Date of publication of application : 23.10.85 Bulletin 85/43

(45) Publication of the grant of the patent : 11.11.87 Bulletin 87/46

(84) Designated contracting states : AT BE CH DE FR GB IT LI LU NL SE

(56) References cited :
CHEMICAL ABSTRACTS, vol. 87, no. 19, 7th November 1977, p. 583, no. 152077q, Columbus, Ohio (US); K.WEGNER et al.: "Imidazole synthesis. Part XI. Synthesis of alpha-substituted 4(5)-methoxymethyl-5(4)-methylimidazoles"

(73) Proprietor : MICROBIAL CHEMISTRY RESEARCH FOUNDATION
14-23, Kamiosaki 3 Chome Shinagawa-ku
Tokyo 141 (JP)

(72) Inventor : Umezawa, Hamao, Prof.
4-23, Toyotamakita Nerima-ku
Tokyo (JP)
Inventor : Ohno, Masaji
1565-13, Koshigoe
Kamakura City Tokyo (JP)

(74) Representative : Becker, Heinrich Karl Engelbert, Dr. et al
HOECHST AKTIENGESELLSCHAFT Central Patent Department P.O. Box 80 03 20
D-6230 Frankfurt am Main 80 (DE)

EP 0 158 843 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to a N-(6-substituted pyridine-2-carbonyl)-3-substituted histidine derivate of the formula

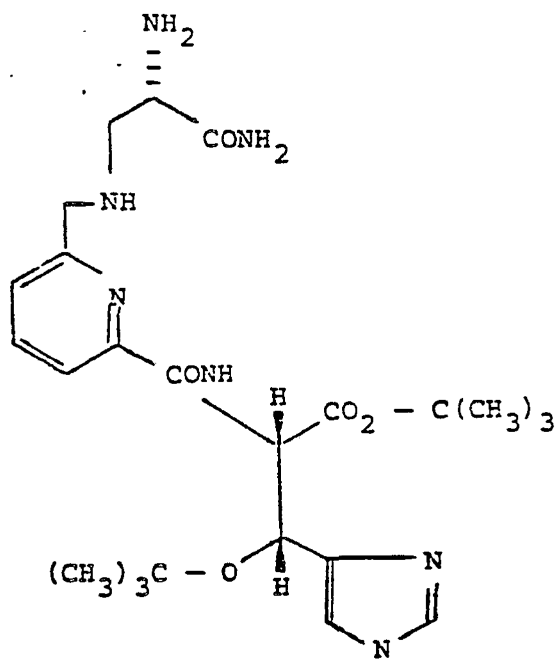

(I)

and its salts and to a method of preparing the same. These compounds have anti-tumor activity.

Various studies on the synthesis of analogues with a pyrimidine moiety which is the active site of Bleomycin which is used as an anti-tumor agent have been made, and, as a result, a new N-(6-substituted pyridine-2-carbonyl)-3-substituted histidine derivative of the formula (I) (hereunder referred to as «PYML 4») has been found which forms a complex with metal ions such as Fe (II) ions and has a remarkably high oxygen activating ability. The new compound is, therefore, expected to be of use as an antitumor agent.

The compound of the formula (I) of the present invention can be prepared by eliminating the protecting groups by a conventional method from a compound of the general formula

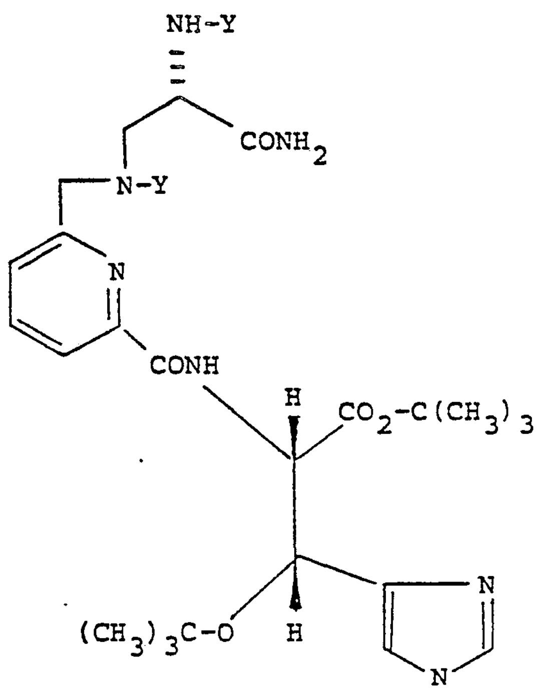

(II)

wherein each Y represents a protecting group.

Any protecting groups used for the protection of an amino group can be used as the protecting groups in the compound of the formula (II) ; a benzyloxycarbonyl or t-butyloxycarbonyl, particularly o-nitrophenylsulfenyl group (hereunder referred to as « Nps ») is preferred.

The elimination of the protecting group can be accomplished by catalytic reduction, hydrolysis, or acid decomposition, depending on the protecting group. When the protecting groups is the Nps group, it can be eliminated by hydrolysis at 0 °C to 60 °C, generally at room temperature, with an organic acid such as citric acid, a halogeno acid such as hydrochloric acid or hydrobromic acid, or an inorganic acid such as sulfuric acid.

The compound of the general formula (II) can be prepared by reacting erythro-3-t-butoxy-L(s)-histidine-t-butyl ester (hereunder referred to as « BHBE ») of the formula (III)

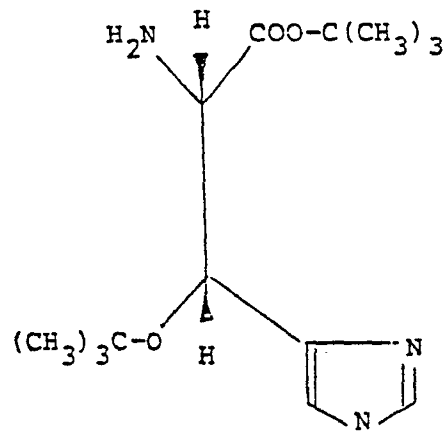

(III)

or its salt with a 6-[[N,N'-diprotected-((s)-2-amino-2-carbamoylethyl) amino] methyl] pyridine-2-carboxylic acid of the formula (IV)

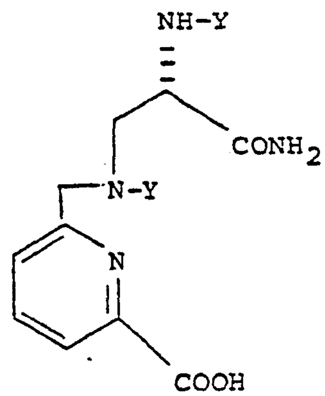

(IV)

wherein Y is as defined above, or its reactive derivative. Any reactive group that is generally used for preparing acid amides can be used as the reactive derivative. Examples of possible reactive derivatives include active esters and acid anhydrides.

The reaction can be carried out in an inert organic solvent at a temperature between — 10°C and the boiling point of the solvent used ; a temperature in the range of 0 °C to 40 °C is usually preferred.

The isolation and purification of the reaction product may be performed by chromatography.

The compound « BHBE » of the formula (III) can be obtained by first protecting the amino groups of erythro-3-hydroxy-L(s)-hystidine (hereunder referred to as « HyHis ») of the formula (V)

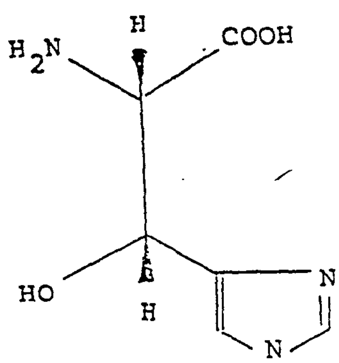

(V)

and then reacting it with isobutene in an inert organic solvent under an inert atmosphere in the presence of an acid catalyst in a sealed tube.

The compound of the general formula (IV) can be prepared in the following manner:

Methyl 6-formylpyridine-2-carboxylate (hereunder referred to as « HPy CME ») is a reacted with (s)-3-amino-2-[tert-butoxycarbonyl) amino] propionic acid amide (hereunder referred to as « Boc-DAPA »). The product is then reduced to form methyl 6-[[(s)-2-Box-amino-2-carbamoylethyl) amino] methyl]-pyridine-2-carboxylate (hereunder referred to as « Boc-DAPAPy ») of the formula VI

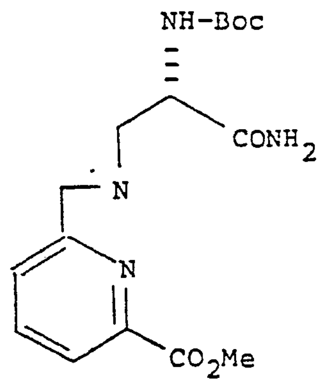

(VI)

and, finally, its imino group is protected by a conventional method to form a compound of the formula (IV).

The measurement of the oxygen activating ability of the compound of the present invention will be described below. The oxygen activating ability was measured by the Spintrap method in the following manner:

Fifty µl of 4 mM aqueous PYML 4, 50 µl of 0.2 M Tris-HCl (pH 8.3) and 60 µl of 14 mg/ml tert-butyl-α-phenyl-nitrogen in ethanol were mixed. To this mixture, 50 µl of 4 mM aqueous $FeSO_4 \cdot 7H_2O$ was added and 30 seconds later, 10 µl of 45 mM aqueous $Na_2S_2O_2$ was added. The ESR was measured at room temperature 3 minutes later.

An ESR measurement was also made by using, as a control, N-[6-[[(s)-2-amino-2-carbamoylethyl) amino] methyl]-pyridine-2-carbonyl]-L-histidine (hereunder referred to as « PYML ») in place of PYML 4.

The results obtained are summarized below.

Table I

Comparison of oxygen activating ability

| Compound | Readings | Signal amplification | Spin concentration | Relative value |
|---|---|---|---|---|
| PYML (Control) | 63.1 | $1.25 \times 10^3$ | 0.05048 | 1 |
| PYML 4 (Compound of the present invention) | 99.3 | $5 \times 10^2$ | 0.1986 | 3.93 |

The table above shows that the compound of the present invention has a remarkably high oxygen activating activity (3.9 times that of PYML). Thus, it is expected that the compound of the present invention will cleave DNA's double helix and exhibit good anti-tumor activity.

The present invention will be further illustrated by the following samples.

Example 1

a) Synthesis of BHBE of the formula (III)

N-benzyloxycarbonyl-BHBE (26.3 mg, 0.0630 mmol) was dissolved in 2 ml of 10 % acetic acid in methanol under an argon atmosphere. To this solution was added 30 mg of Pd-black and the mixture was refluxed for 2 hours. After agitating at room temperature for another one hour, the Pd-black was filtered

off. The filtrate was concentrated at below 30 °C. After a substantial amount of methanol was distilled off, 10 ml of heptane was added below 30 °C to cause azeotropic distillation of formic acid. After repeating this procedure four times, 2 ml of toluene was added to the concentrated residue and the mixture was concentrated to dryness. To the solid residue was added 1 ml of 0.3 N HCl to make a solution, which was concentrated at below 30 °C. Azeotropic distillation with 5 ml of toluene was done three times and the residue concentrated to dryness to give BHBE dihydrochloride as a white foam.

b) Synhesis of the compound of the general formula (IV)

HPyCME (658.3 mg, 2.96 mmol) was dissolved in 20 ml of $CH_3CN$ under an argon atmosphere. To this solution were added 3.5 g of an activated molecular sieve 3A and 20 ml of Boc-DAPA (601.6 mg, 2.96 mmol) in $CH_3CN$ and the mixture was vigorously stirred at room temperature. After standing overnight, the molecular sieve 3A was filtered off through Celite and the filtrate was concentrated to give about 1 g of a yellow foam of Schiff base. Without purification, the base was dissolved in 50 ml of methanol and reacted with hydrogen fed at atmospheric pressure in the presence of 100 mg of 10 % Pd/C for three hours. The Pd/C was filtered off and the filtrate was concentrated, after which the residue was purified by column chromatography over silica gel ($CH_3OH/CH_2Cl_2$ = 1/19) to give Boc DAPAPy as a pale yellow foam. The product (120 mg, 0.341 mmol) was dissolved in 3 ml of $CH_2Cl_2$ and to this solution, 3 ml of TFA was slowly added dropwise at 0 °C. The mixture was stirred at 0 °C for 10 minutes, then at room temperature for another 50 minutes. The reaction mixture was concentrated and the residue was dissolved in 5 ml of $CH_2Cl_2$. To this solution, 0.214 ml of triethylamine (4.5 equivalents) and 84.4 mg of Nps-Cl (1.3 equivalents) were added at 0 °C and stirred at room temperature overnight. The reaction mixture was diluted with 10 ml of $CH_2Cl_2$ and the organic layer was washed with 15 ml of water. The washed organic layer was dried over anhydrous sodium sulfate and concentrated. The residue was purified by column chromatography over silica gel (AcOEt) to yield 121.9 mg (0.218 mmol) of methyl 6-[[(s)-2-Nps-amino-2-carbamoylethyl)-Nps-amino] methyl] pyridine-2-carboxylic acid (hereunder referred to as « N,N-diNpsACAPyC »).

$[\alpha]_D^{22}$ + 13.4 (C 1.0, $CHCl_3$)

SIMS m/z = 561 ($M^+$ + 1), 407 ($M^+$ — Npys)

$^1$HNMR ($CDCl_3$, TMS) δ 3.36 — 3.74 (2H, m), 3.89 (3H, s), 3.90 — 4.04 (1H; m), 4.80 (2H, s), 5.24 (1H, br, s), 5.67 (2H, s), 7.16 — 8.96 (9H, m)

IR ($CHCl_3$) 3 510, 3 470, 2 990, 1 725, 1 680, 1 585, 1 510, 1 435, 1 335 $cm^{-1}$.

To a solution of 101.1 mg (0.180 mmol) of the compound in 4 ml of $CH_3OH$, 3 ml of 0.1 N LiOH was added dropwise at 0 °C and the mixture was stirred at room temperature for 5 hours. To this mixture, water was added and MeOH was distilled off. The residue was washed with 5 ml of AcOEt and the aqueous phase was acidified with citric acid and extracted with AcOEt. The organic phase was washed with water to remove traces of citric acid. The organic phase was dried over anhydrous sodium sulfate and concentrated to give 84.2 mg of N,N-DiNpsACAPyC as a yellow powder.

c) Synthesis of the compound of the general formula (II)

All of the BHBE obtained in (a) above and 24.7 mg of N,N-diNpsACAPyC obtained in (b) above were suspended in 3 ml of $CH_3CN$ under an argon atmosphere and 22 μl of triethylamine and 21 μl of DPPA were added in that order and reacted at room temperature overnight. The reaction mixture was purified by TLC using the developing system of AcOEt : EtOH : $H_2O$ (3 : 1 : 1) to give 23.3 mg (0.0287 mmol) of a yellow oil of the compound of the general formula (II) wherein Y is Nps.

$[\alpha]_D^{23}$ + 39.2 (C 0.8, $CHCl_3$)

SIMS m/z = 812 ($M^+$), 658 ($M^+$ — Npys), 602 ($M^+$ — Npys — $Bu^t$)

$^1$HNMR ($CDCl_3$, TMS) δ 1.23 (9H, s), 1.48 (9H, s), 3.44 — 3.78 (2H, br, s), 380 — 3.96 (1H, m), 4.64 — 4.84 (1H, m), 4.75 (2H, s), 4.96 — 5.16 (1H, m), 5.28 (2H, s), 6.60 (1H, br, s), 7.00, (1H, s), 7.20 — 8.90 (10H, m)

IR ($CHCl_3$) 3 450, 2 970, 1 730, 1 675, 1 585, 1 512, 1 335, 1 155 $cm^{-1}$.

d) Synthesis of PYML 4

A portion (2.1 mg, 2.59 × $10^{-3}$ mmol) of the compound of the general formula (II) obtained in (c) above was dissolved in 0.2 ml of methanol. To the solution, 5 mg (excess amount) of 3-methylindole was added and 0.4 ml of 0.2 N HBr was added dropwise and the mixture was stirred at room temperature overnight. The reaction mixture was partitioned between 2 ml of water and 2 ml of ethyl acetate and the aqueous phase was washed two more times with 1-ml portion of ethyl acetate. The aqueous layer was passed through 0.5 ml of an ion exchange resin, Diaion$^R$ WA30 ($^{\ominus}$OH form) and neutralized. The effluent was concentrated to dryness to give 3.7 mg of pale yellow powder, to which 1 ml of ethyl acetate was added to extract the yellow component. After drying, 2.8 mg of a white powder was obtained. This white powder was dissolved in 1 ml of water and passed through 0.5 ml of an ion exchange resin, Diaion$^R$ SA 10A ($^{\ominus}$OH form) and then washed with another 3 ml of water. The fractions which were positive in both

ninhydrin and Pauly's reactions at 250 nm (UV) were collected and concentrated to dryness to afford 0.8 mg ($1.59 \times 10^{-3}$ mmol) of a white powder. Yield : 61.3 %

Rf = 0.83 (npPrOH : Pyridin : $H_2O$ : AcOH = 15 : 10 : 12 : 3),

0.41 (10 % $NH_{(40Ac\ :\ MeOH\ =\ 1\ :\ 1)}$

Ninhydrin reaction : +

Pauly's reaction : +

$[\alpha]_D^{23}$ + 4.3 (C 0.1, $CH_3OH$)

$^1$HNMR (D 0, Acetone · δ 2.19) δ 1.19 (9H, s)

WEFT 7.17 (1H, s), 7.47 — 8.03 (4H, m)

ESR (Cu(II) complex, Mn(II)/MgO $_{\Delta}H_{3\text{-}4}$ 86.9 G)

$g_{11}$ 2.225, $g_1$ 2.053, A(G) 185.0

IR (KBr) 3 360, 2 955, 2 910, 1 735, 1 660, 1 595, 1 460, 1 365, 1 155, 1 080 $cm^{-1}$.

Note : ESR was measured on a complex of the compound of the general formula (I) with copper ion.

Referential Example 1

Synthesis of N-benzyloxycarbonyl-BHBE

To a suspension of 82.3 mg (0.481 mmol) of erythro-3-hydroxy-L(s)-histidine in 2 ml of water, 80.5 μl (1.2 equivalents) of triethylamine and a solution of benzyloxycarbonyl chloride (158.5 mg, 1.2 equivalents) in dioxane (2 ml) were added in that order at 0 °C and the mixture was stirred at room temperature overnight, after which 5 ml of water was added and the mixture was partitioned with 10 ml of diethyl ether. The aqueous phase was concentrated to dryness and the residue was dissolved in methanol and then transferred into a sealed tube. Methanol was purged with nitrogen and the contents of the tube were concentrated in vacuo to dryness. To the residue, 2 ml of $CH_2Cl_2$ was added and isobutene was blown into the mixture at — 78 °C until the volume of the mixture was 1.5 times its initial volume, after which five drops of conc. $H_2SO_4$ were added and the mixture was stirred at room temperature for 48 hours. The reaction mixture was diluted with 5 ml of $CH_2Cl_2$ and vigorously agitated with 10 ml of saturated $NaHCO_3$ at 0 °C for 30 minutes. After washing the organic layer with water, it was dried over anhydrous sodium sulfate and concentrated. The residue was purified by column chromatography over silica gel (30 times the weight of the residue ; AcOEt → MeOH/$CH_2Cl_2$ = 1/9) to give 71.5 mg (0.171 mmol) of N-benzyloxycarbonyl-BHBE as a colorless oil.

$[\alpha]_D^{18}$ + 36.6 (C 1.3, $CHCl_3$)

SIMS m/z = 418 ($M^+$ + 1), 362 ($M^+$ — $Bu^t$), 306 ($M^+$ — 2 × $Bu^t$)

$^1$HNMR ($CDCl_3$, TMS) δ 1.18 (9H, s), 1.41 (9H, s), 4.52 (1H, dd, J = 4.0, 8.5 Hz), 5.03 (1H, d, J = 4.0 Hz), 5.09 (2H, s), 5.80 (1H, d, J = 8,5 Hz), 6,90 (1H, s), 7.13 (5H, s), 7.54 (1H, s)

IR ($CHCl_3$) 3 430, 2 970, 1 720, 1 510, 1 150 $cm^{-1}$.

**Claims** (for the Contracting States : BE, CH, DE, FR, GB, IT, LI, LU, NL, SE)

1. A compound of the formula I

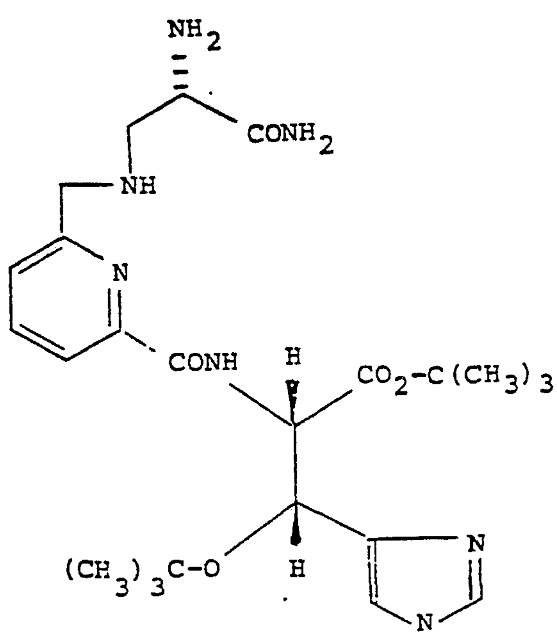

(I)

or a physiologically acceptable acid addition salt.

2. A method of preparing a compound of the formula I

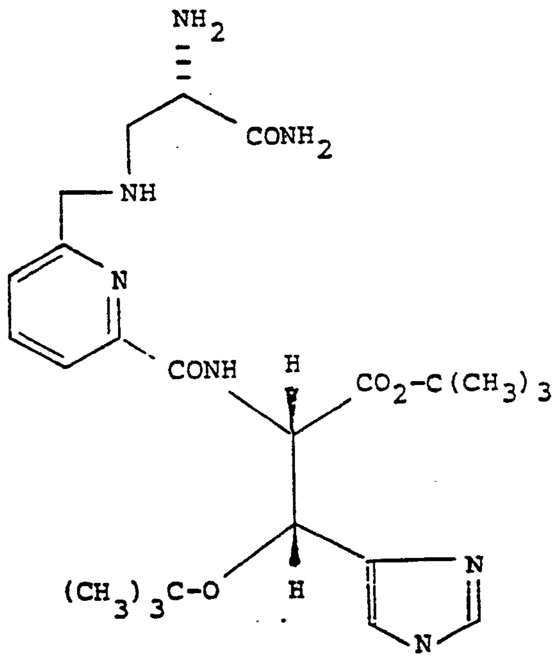

(I)

which comprises eliminating by a conventional method the protecting groups from a compound of the formula II

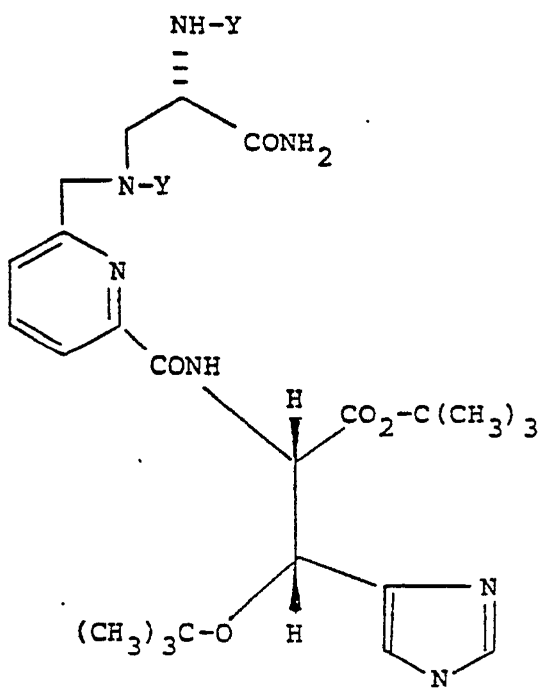

(II)

wherein Y represents a protective group, and optionally prepare in a conventional manner a physiologically active acid addition salt.

**Claim** (for the Contracting State AT)

A method of preparing a compound of the formula I

(See formula page 8)

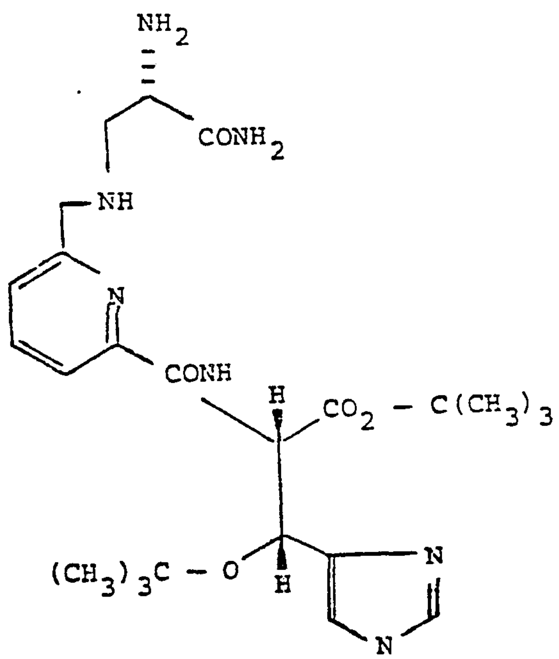

(I)

which comprises eliminating by a conventional method the protecting groups from a compound of the formula II

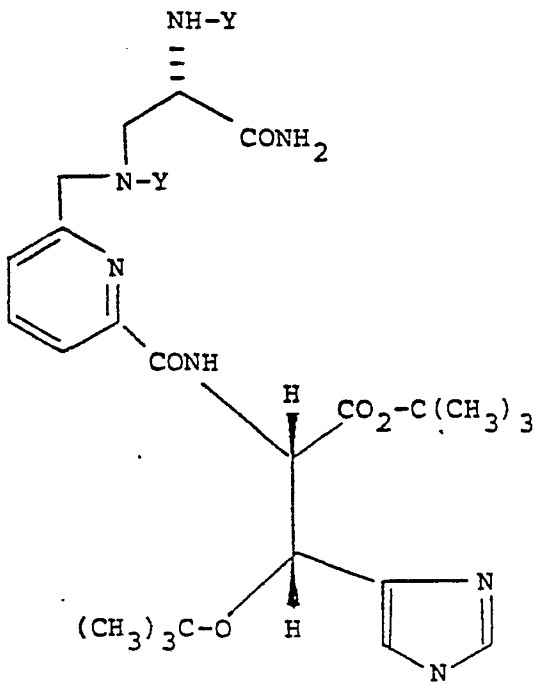

(II)

wherein Y represents a protective group, and optionally prepare in a conventional manner a physiologically active acid addition salt.

**Patentansprüche** (für die Vertragsstaaten : BE, CH, DE, FR, GB, IT, LI, LU, NL, SE)

1. Verbindung der Formel I

(Siehe Formel I Seite 9 f.)

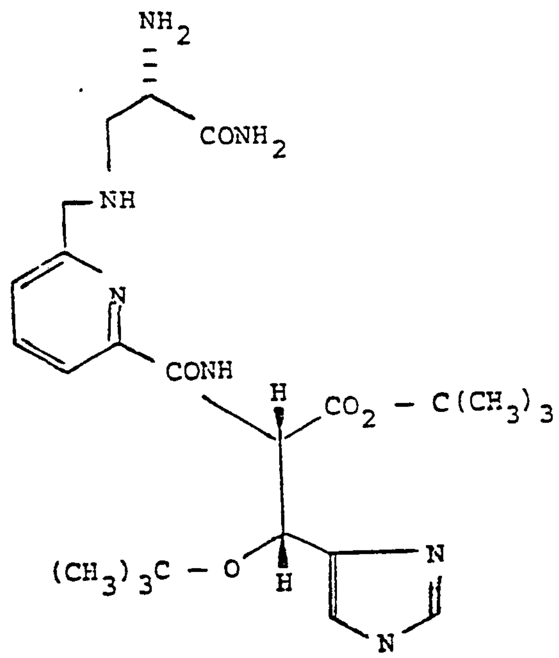

(I)

bzw. deren physiologisch unbedenkliche Säureadditionssalze.

2. Verfahren zur Herstellung der Verbindung der Formel I

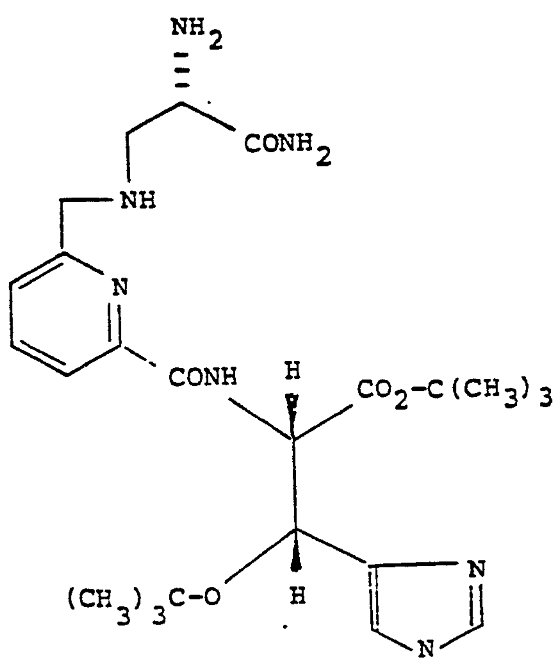

(I)

dadurch gekennzeichnet, dass man aus einer Verbindung der Formel II

(Siehe Formel II Seite 10 f.)

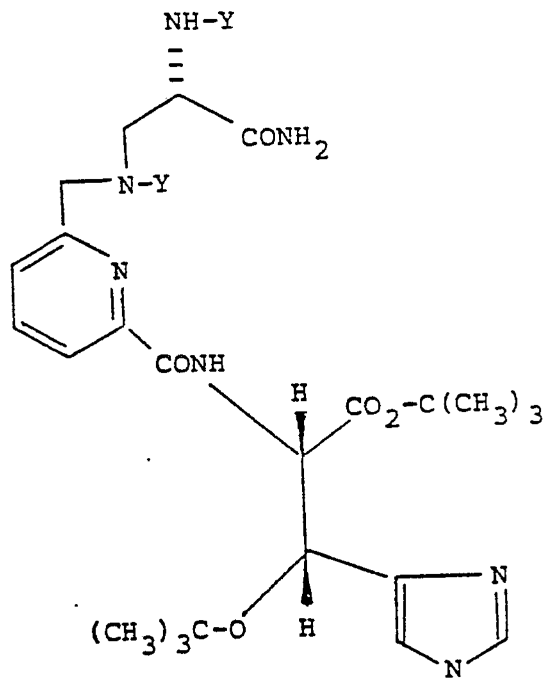

(II)

worin Y für eine Schutzgruppe steht, nach einer üblichen Methode die Schutzgruppen abspaltet und gegebenenfalls auf übliche Weise ein physiologisch wirksames Säureadditionssalz bildet.

**Patentanspruch** (für den Vertragsstaat AT)

Verfahren zur Herstellung der Verbindung der Formel I

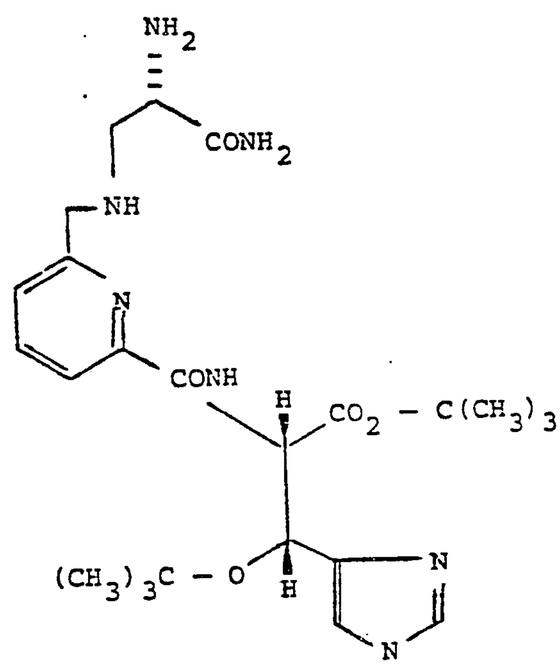

(I)

dadurch gekennzeichnet, dass man aus einer Verbindung der Formel II

(Siehe Formel II Seite 11 f.)

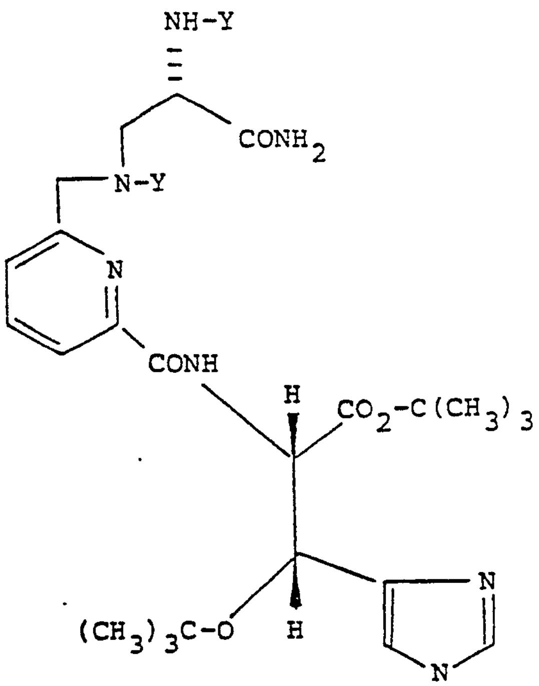

(II)

worin Y für eine Schutzgruppe steht, nach einer üblichen Methode die Schutzgruppen abspaltet und gegebenenfalls auf übliche Weise ein physiologisch wirksames Säureadditionssalz bildet.

**Revendications** (pour les Etats contractants : BE, CH, DE, FR, GB, IT, LI, LU, NL, SE)

1. Composé de formule I

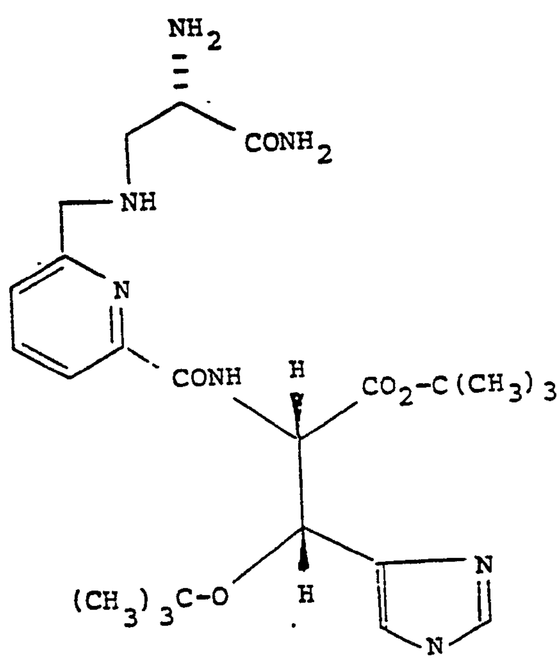

(I)

ou un sel d'addition avec un acide physiologiquement acceptable.

2. Procédé de préparation d'un composé de formule I :

(Voir formule I page 12)

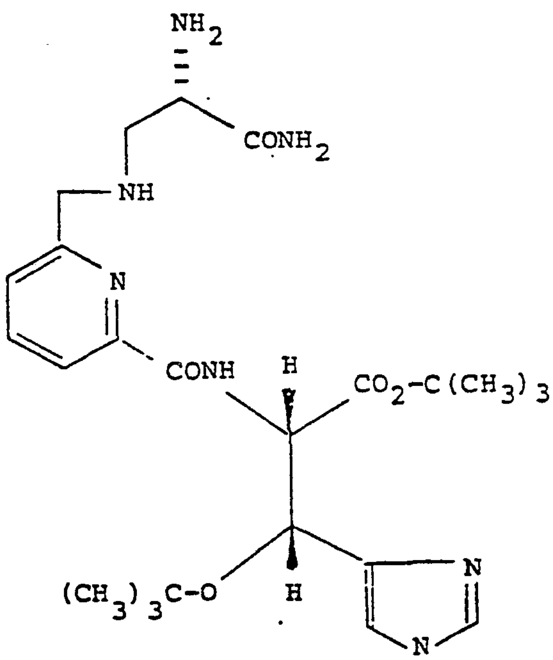

(I)

qui comprend l'élimination par une méthode classique des groupes protecteurs d'un composé de formule II :

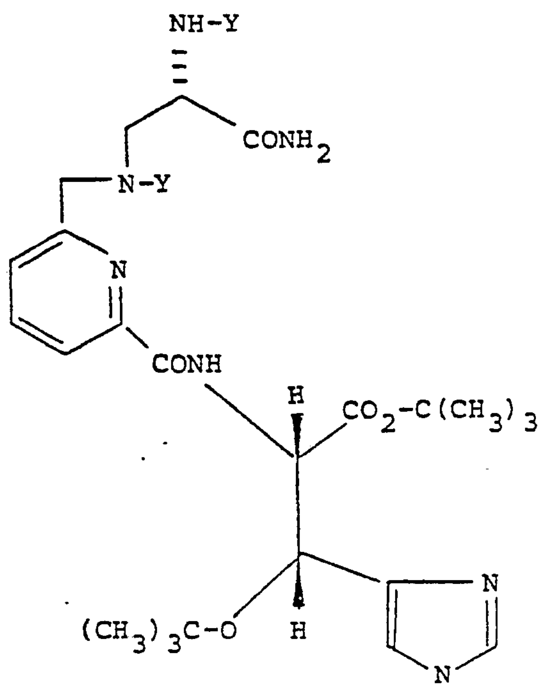

(II)

dans laquelle Y représente un groupe protecteur et éventuellement la préparation d'une manière classique de sels d'addition avec un acide physiologiquement actif.

**Revendication** (pour l'Etat contractant AT)

1. Procédé de préparation d'un composé de formule I :

(Voir formule I page 13)

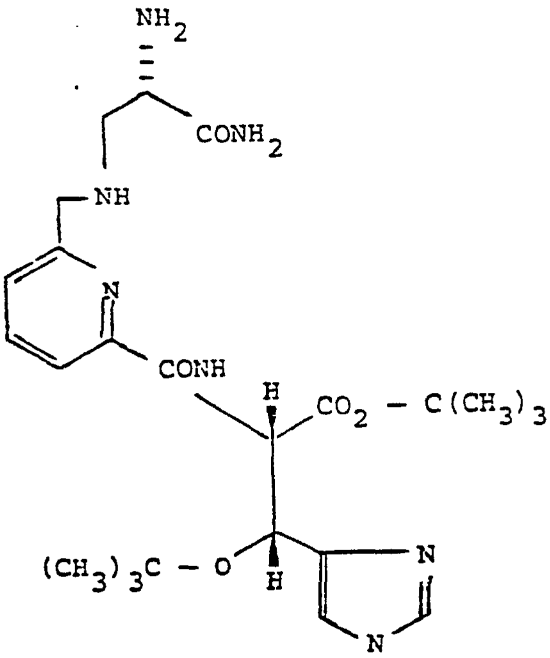

(I)

qui comprend l'élimination par une méthode classique des groupes protecteurs d'un composé de formule II :

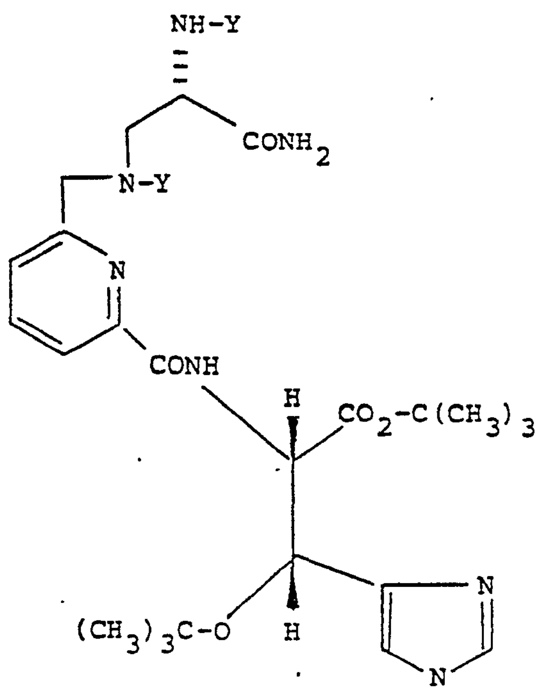

(II)

dans laquelle Y représente un groupe protecteur et éventuellement la préparation d'une manière classique de sels d'addition avec un acide physiologiquement actif.